# EUROPEAN PATENT APPLICATION

(11) **EP 2 189 183 A1**
(43) Date of publication of application: **26.05.2010**
(21) Application number: 10153813.0
(22) Date of filing: 05.10.2006
(51) Int. Cl.: A61N 5/06

(54) **Apparatus for sublingual application of light to blood**

(30) Priority: 12.10.2005 US 248995
(62) Divisional of application: 06825640.3
(71) Applicant: Perez, Thomas, Chicago, IL 60618 (US)
(72) Inventor: Perez, Thomas, Chicago, IL 60618 (US)
(74) Representative: Ruschke, Hans Edvard

(57) **Abstract**

Light having one or more therapeutic wavelengths is applied to a patient's blood while that blood remains in the body. The UV light is applied sublingually. A shell having a cold cathode fluorescent bulb is inserted under a patient's tongue to irradiate the mucus membrane and provide the UV light to the blood.

## Description

This application is a continuation in part of US Application No. 11/235652 filed 26 September 2005 that is a is a continuation in part of US Application No. 11/140272 filed 27 May 2005 that is a continuation in part of US Application Number 11/076169 filed 9 March 2005 and US Application Number 10/926209 filed 25 August 2004 that claimed the benefit of US Provisional Application Number 60/503,678 filed September 17, 2003.

### BACKGROUND OF THE INVENTION

### Field of the Invention

The present invention relates to a method and apparatus for providing light to blood. More specifically, the present invention relates to a method and apparatus for sublingual irradiation.

### Background of the Prior Art

Ultraviolet (UV) light can be used to treat a multitude of medical problems, including for example bacterial, viral and fungal infections, poisoning, fatigue, Alzheimer's disease, allergies and asthma, rheumatic diseases and arthritis, diabetes, hepatitis, and cancer. UV light sterilizes the blood and acts as an antibiotic.

The UV light is applied either to the patient's skin or directly to the blood. If the UV light is applied to the skin it is typically provided to the patient's skin either with a wrap or lamp.

UV light is commonly used to treat jaundiced babies. Because infant's skin is thin and the blood vessels are close to the surface, UV light is typical applied to the skin when treating jaundiced babies.

Applying the UV light directly to a patient's blood supply is known as photoluminescence or UV blood illumination (UBI). UV blood illumination increases oxygen, destroys toxins and boosts the Immune system.

In prior art UBI, a small amount of blood is drawn from the patient, up to about 250 cc. The body has about 5.6 L of blood. The blood that is drawn travels through a cuvette or glass chamber. The blood is repeatedly illuminated with UV light and then returned to the body. The process is repeated, typically a day or several days later.
These treatments are time consuming, and require regular trips to a medical facility. In addition, trained personal must be available to provide the treatments.

There is a need for a method of providing UV light to a patient's entire blood supply, not just a small portion of it. There is a need for a system that is convenient for the patient, which does not require regular doctor visits. There is a need for a simple system that can be used by the patient in his home.

There is a need for a system that allows for round the dock treatments or other regular treatments such as pulsed treatment or automatic periodic treatments.

There is a need for a blood illuminator that reduces the risk of infection from removing blood. There is a need for a system that reduces the number of needle sticks a patient must endure.

There is a need for a system that allows the blood to be treated on an as needed basis, such as based on how the patient is feeling at a particular time.

### SUMMARY OF THE INVENTION

The present invention is a light device or a portable light pack that irradiates the mucous membrane under the tongue. The light pack has a battery or other power supply and a light source. The light source emits light at one or more therapeutic wavelengths. Preferably, the light is UV light at one or more therapeutic wavelengths. The UV light source is typically LEDs that emit UV-A or UV-C light or a combination of UV-A and EJV-C light. The light pack or device is inserted into the patient's mouth, preferably under the tongue. Fiber optic strand(s) run through a tube to illuminate the mucous membrane under the tongue. Capillaries are plentiful and close to the surface under the tongue, thus illuminating the blood. A shell having a cold cathode fluorescent bulb is inserted under a patient's tongue to irradiate the mucus membrane and provide the UV light to the blood.

### BRIEF DESCRIPTION OF THE DRAWINGS

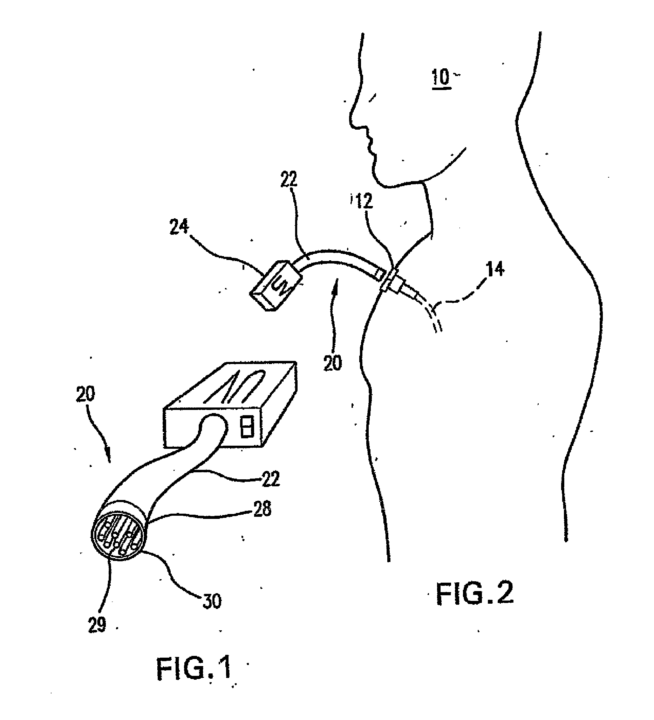

Figure 1 is a portable light pack;
Figure 2 is a patient with a connectable light pack;

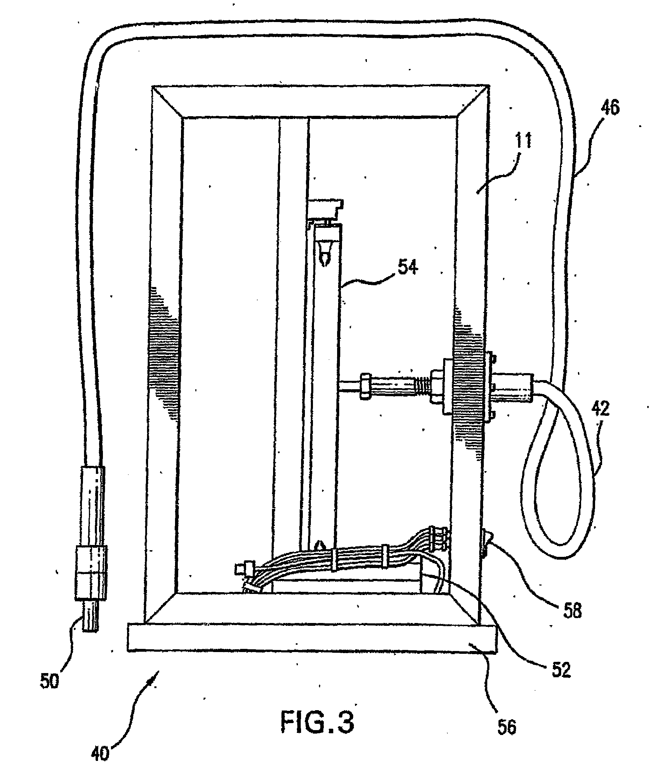

Figure 3 is a cross section of a light device;

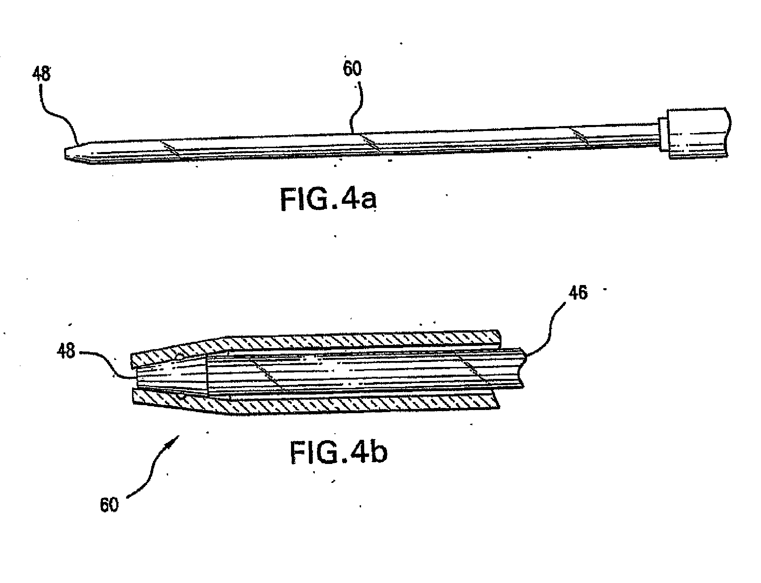

Figure 4a is a UV catheter for use with a light pack or with a light device;
Figure 4b is a cross section of the light catheter for use with a light pack or with a light device;

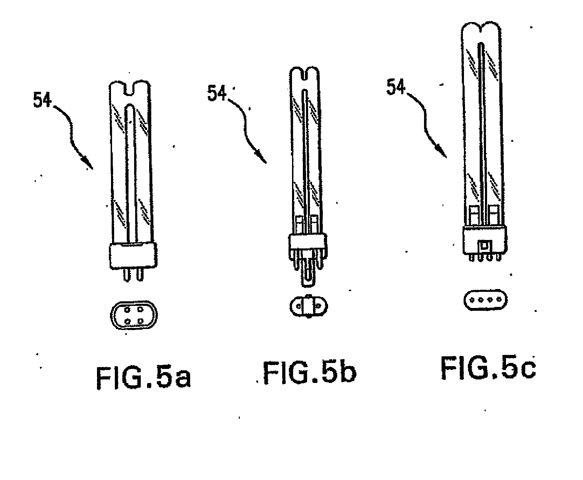

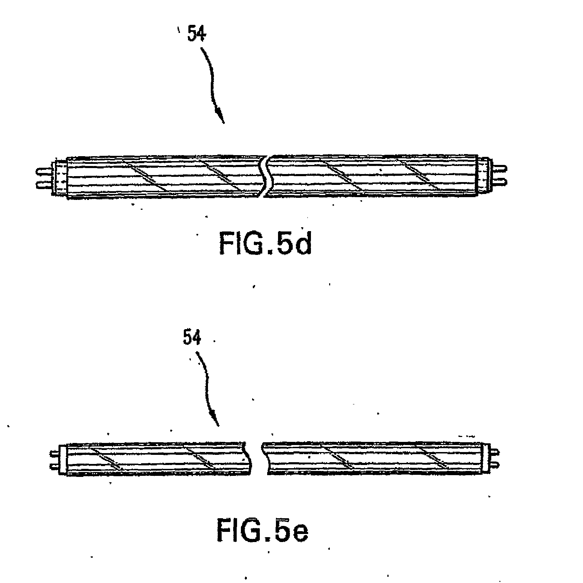

Figures 5a-e are various embodiments of the UV light bulb for with the light pack or with the light device.

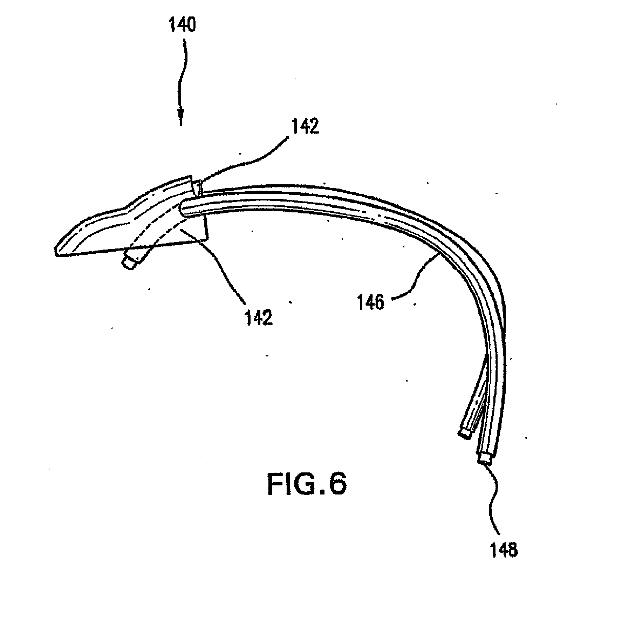

Figures 6 is a sub-lingual light irradiation delivery device.
Figure 7 is the sub-lingual light irradiation delivery device inserted into a patient's mouth.
Figure 8 is an alternative embodiment of the sub-lingual light irradiation delivery device.
Figure 9 is an alternative embodiment of the sub-lingual light irradiation delivery device.
Figures 10a and 10b are alternative embodiments of the sub-lingual irradiation delivery device.
Figures 11a-c are an alternative cold cathode fluorescent bulb for use with the sublingual irradiation delivery devices of Figures 9, 10a and 10b.

### DETAILED DESCRIPTION OF THE INVENTION

Light at one or more therapeutic wavelength, such as ultraviolet light (UV), is used to treat many diseases including infections, poisoning, fatigue, allergies, hepatitis, cancer and HIV. UV light increases the oxygen combining power of the blood, destroys toxins, viruses, fungl, bacteria, and boosts the immune system. UV light also sterilizes the blood and acts as an antibiotic. Preferably, UV light at one or more therapeutic wavelength is utilized in the present invention. More preferably the light is either UV-A or UV-C light is utilized in the present invention. For some conditions and/or diseases UV-A light is more effective than UV-C and for other conditions and/or diseases UV-C light is more effective than UV-A light. The wavelengths or wavelengths of light to be used to treat the patient are selected based on the wavelength or wavelength that will best treat the condition or disease of the patient.

The invention is a light device 40 and a portable light pack 20 that are connectable to a patient 10 via a port 12 to directly supply light at a therapeutic wavelength(s), preferably UV light, to the patient's blood supply 14. Port 12 is surgically implanted in patient 10. Ports and catheters are well known in the art. They are for cancer patients receiving regular or continuous chemotherapy, diabetics and others.
Alternatively, light pack 20 could be surgically implanted under the patient's skin. In yet another alternative, a portion of light pack 20 such as a portion of light guide 22 can be implanted in patient 10.

Portable light pack 20 comprises housing 24, battery or other power source (not shown) and light source (not shown). Preferably, light source (not shown) is LEDs (not shown) emitting UV light at a therapeutic wavelength(s). More preferably, the light is UV-C light, UV-A light or a combination thereof. Alternatively, a plurality of LEDs, having one or more different wavelengths of light at one or more therapeutic wavelengths be used. It is preferable that a substantial portion of the emitted light be UV-C and/or UVA.

In one embodiment, light pack 20 has a light guide 22 made of rubber or other flexible tubing for housing one or more fiber optic strands 26. Alternatively, a liquid core light guide or other known light guide can be used. Emitted radiation travels to the end tip of the light guide 22 and is emitted. Emitted radiation directly illuminates patient's blood. Light guide 22 has transparent cover 28 at the end connectable to or insertable in the patient.

Light guide 22 has a connector 30 for coupling light pack 20 to port 12 or catheter in the patient. Catheter may be inserted into port 12 or there may be connector 30 on one end of the flexible tube that mates with a connector on port 12.

In an alternative embodiment, light source such as a LED, or miniature light bulb is inserted through port 12 and directly illuminates the blood.

The light pack 20 allows therapeutic wavelength(s) of light to be supplied directly to the blood. Instead of treating only a maximum of 250 cc of blood, larger amounts of blood or even the entire blood supply can be treated. The 5,6 L of blood in a human body circulates through the body about 3 times every minute. Thus, large amounts of blood can be treated with photoluminescence.

Patient 10 can connect to light pack 20 when a treatment is needed. Alternatively, light pack 20 can remain connected to port 12 and be turned on only for treatment. Light pack 20 could be turned on and off manually. Alternatively, light pack 20 could automatically turn off the light source after a set treatment time, such as 20 minutes. Light pack 20 could have a controller such as a computer or other smart interface that limits the number of treatments given time period, limits the total amount of treatment time in a given time period, automatically provides treatments, pulses the light source, or provides only particular wavelengths. The computer or other smart interface could keep a treatment record. The computer or other smart interface could communicate wirelessly, via the Internet or through other electronic means to automatically update the doctor's treatment records. Computer preferably can automatically adjust treatment time, wavelength or other factors based on patient input, doctor orders or other data.

In an alternative embodiment, light pack 20 or a portion of light pack 20, such as an end of light guide 22 is surgically attached to patient 10 or implanted in patient 10.

Light device 40 can be attached to patient 10 via port 12 to directly illuminate the blood. Light device 40 comprises housing 44, light guide 42, and light source 54 adapted to emit radiation at one or more therapeutic wavelengths. Preferably, the light source is a UV light source 54 such as a medical grade UV light bulb. Light source 54 preferably emits light in the UV-C, UV-A or UV-A and UV-C range. Housing 44 preferably has a weighted base 56. There are preferably electronics 52 such as a power supply or power cord for connection to a power source. Light device preferably has a manual on/off switch 58. Electronics 52 also preferably include a controller, a timer or smart interface such as a computer.

Catheter 60 with light guide 42 is inserted into port 12 to directly illuminate the blood. Light guide 42 may have connector 50 that mates with a connecter on port 12. Light guide 42 may be one or more fiber optic strands in a flexible tube. Alternatively, light guide 42 may be a liquid core light guide 46 or other known light guide. In yet another alternative, light source is a LED or small light bulb at the end of a flexible tube adapted to be inserted through port 12 to directly illuminate the blood.

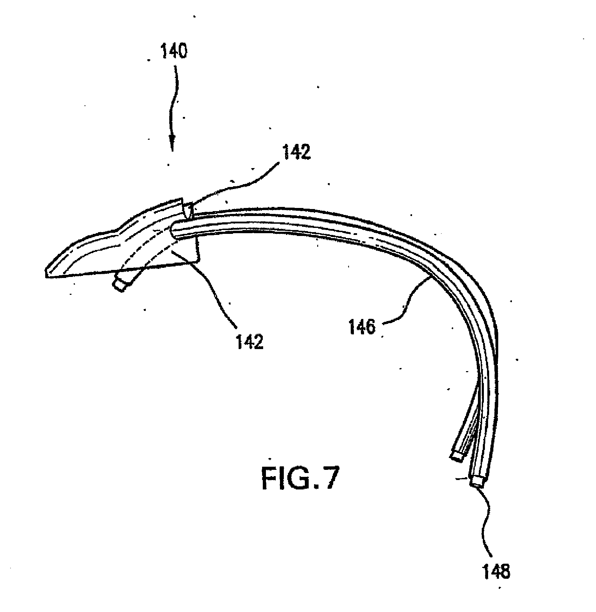

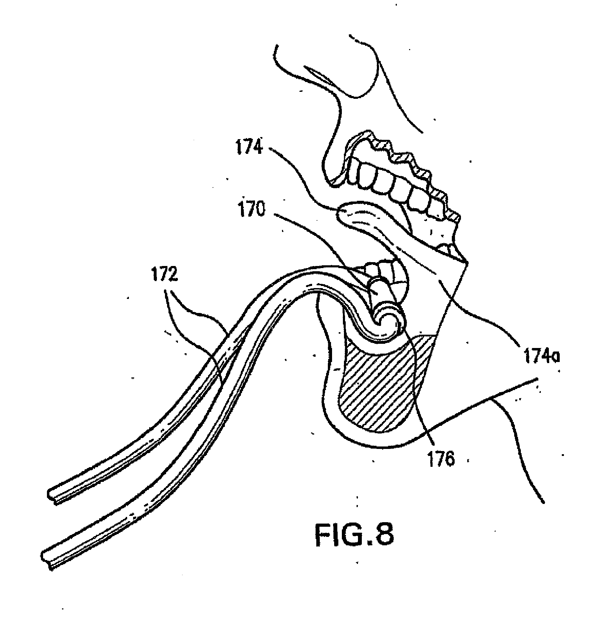

In another embodiment of the invention, as shown in Figures 7 and 8, light at a therapeutic wavelength is administered under the tongue. The capillaries under the tongue are close to the surface. These capillaries are very sensitive. Capillary exposure of the mucus membrane is significantly greater than other exposed body surfaces. The greater capillary exposure allows for greater penetration of the ultraviolet spectrum. The Light device 140 comprises a mouthpiece 142 for holding and aligning the light source under the tongue. Mouthpiece 142 is inserted into patient's mouth under the tongue.
The mouthpiece 142 has at least one aperture 144 through which tubing 146 is inserted. Tubing 146 is preferably plastic tubing and is preferably flexible. The tubing may be adjustably inserted through the at least one aperture 144 to allow for individual adjustment by the patient 10 or doctor to a preferred treatment location under the patient's tongue. Alternatively, tube 146 can be mounted in the preferred position such that each time the mouthpiece 142 is used, the light is administered at the same location. Preferably, mouthpiece 142 is molded to the shape of patient's 10 mouth. There are preferably fused silica fiber optic bundles 148 in the tubing 144. Fused silica fiber optic bundles 148 are preferred because they do not emit any heat. Optionally, the light source is a cold cathode fluorescence bulb. One or more low voltage cables are used to power the cold cathode fluorescent bulb. The fiber optic bundles 148 preferably deliver UV light at a therapeutic wavelength sublingually. The light is preferably UV-A, UV-C or a combination thereof. In yet another alternative, light source is a LED or small light bulb at the end of the flexible tube adapted to directly irradiate the mucus membrane under the tongue. This delivery system is preferred for relatively young patients without a life threatening disease.

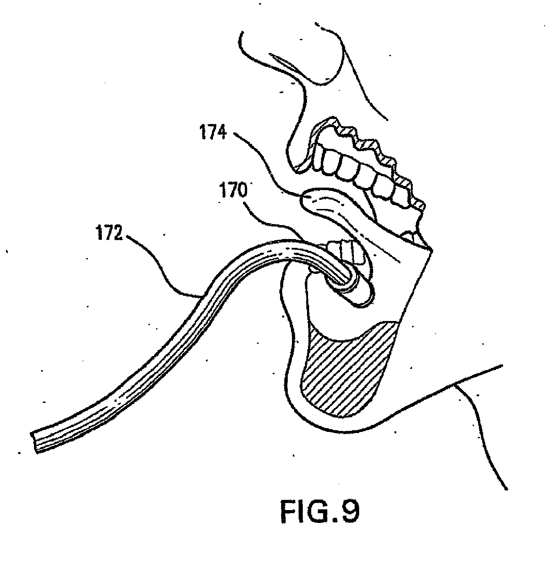

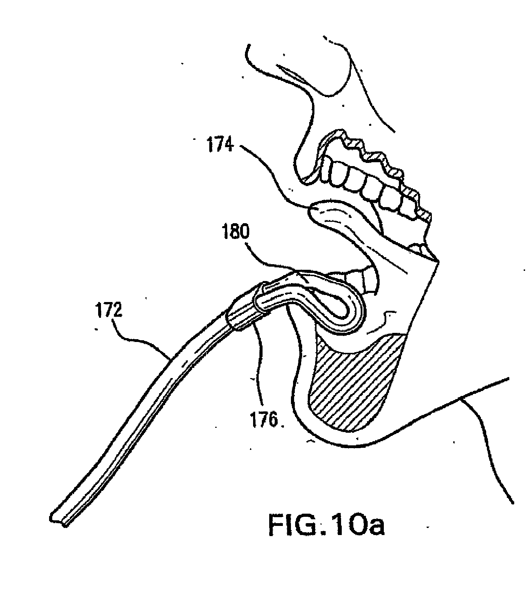

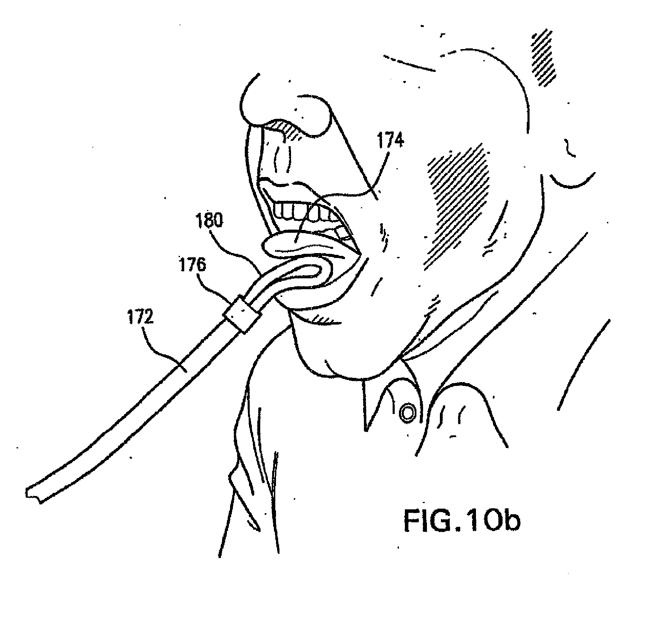

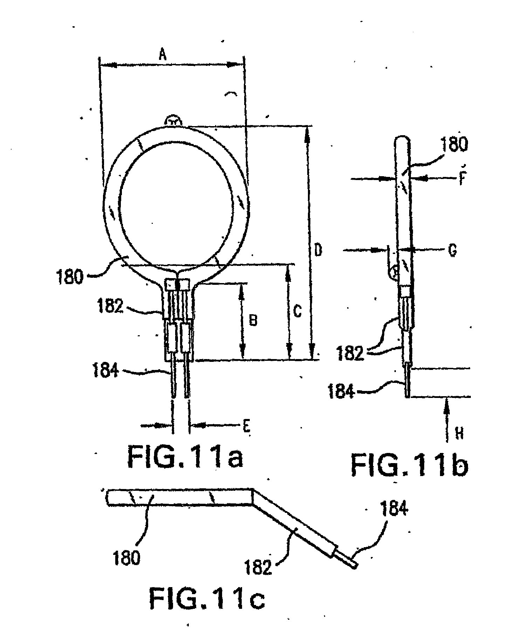

In other embodiments of the invention, as shown in Figures 9 -11, light at a therapeutic wavelength is administered under the tongue to irradiate the capillaries under the tongue. Light device 140 comprises a cold cathode fluorescent bulb or other bulb such as cold fusion bulb. The bulb can be housed in a waterproof shell 170 or the bulb may be adapted to be placed directly under a patient's tongue. The waterproof shell 170 can be oriented in substantially parallel alignment with the back of the tongue 174 or can be aligned substantially perpendicular to the back 174a of tongue 174. The shell 170 is placed under tongue 174 so that the mucous membrane where the capillaries are close to the surface receives the irradiation. Preferably, the shell 170 is made of a material which allows emission of UV light. Optionally, the shell 170 has a window the permits the emission of the UV radiation. If the shell 170 has a window, it is preferable that the window be oriented toward the bottom of the mouth. One or more low voltage cables 172 used to power the cold cathode fluorescent bulb. The low voltage cables are preferably attached to the shell at a waterproof connection 176. The bulb may have a shaft with one or more pins adapted to mate with the low voltage cable with a female connection end. Other known electrical connections can be utilized. It is preferable that the light source be removable for cleaning, sterilization and/or replacement of the bulb. In another alternative embodiment, the bulb and low voltage cable are an integral unit. In yet another alternative embodiment, the bulb and power supply are an integral unit. The light is preferably UV-A, UV-C or a combination thereof.

The light source preferably has a loop shape. The light source also preferably has an angle to make it more comfortable for the patient.

Optionally, shell or bulb 170 is made of a flexible material. This will be more comfortable for the patient. Optionally, the flexible material allows the shell or bulb to mold to the patient's mouth.

Light device 40, 140 allows light at one or more therapeutic wavelengths to be supplied directly to the blood. Instead of treating only a maximum of 250 cc of blood, larger amounts of blood or even the entire blood supply can be treated. The 5.6 L of blood in a human body circulates through the body about 3 times every minute. Thus, large amounts of blood can be treated with photoluminescence.

Patient 10 can connect to the light device 40, 140 when a treatment is needed.
The light device 40, 140 could be turned on and off manually. Alternatively, light device 40, 140 could automatically turn off the light source after a set treatment time, such as 20 minutes. Light device 40, 140 could have a controller, computer or other smart interface that limits the number of treatments given time period, limits the total amount of treatment time in a given time period, automatically provides treatments, pulses the LEDs, or provides only particular wavelengths if the light pack has LEDs of various wavelengths. The computer or other smart interface could keep a treatment record. The computer or other smart interface could communicate wirelessly, via the Internet or through other electronic means to automatically update the doctor's treatment records. The computer could automatically adjust the treatment time based on input from the patient, the doctor, treatment records, or other data.

The following paragraphs 1-21 describe further embodiments of the invention:
1. A blood illuminator comprising: a power supply; a light source powered by said power supply; said light source adapted to emit radiation at one or more therapeutic wavelengths and said light source adapted to be placed under a patient's tongue.
2. The blood illuminator of paragraph 1 wherein the light source is a cold cathode fluorescent bulb.
3. The blood illuminator of paragraph 2 wherein the light source emits UV radiation.
4. The blood illuminator of paragraph 3 wherein the UV radiation is UV-A radiation, UV-C radiation or a combination thereof.
5. The blood illuminator of paragraph 2 wherein the light source is a medical grade UV light bulb.
6. The blood illuminator of paragraph 1 wherein the light source comprises a loop member and a shaft, said shaft electrically, connected to the power supply transparent and a second end portion is connected to the power supply.
7. The blood illuminator of paragraph 6 wherein a low voltage cable connects the power supply and the light source.
8. The blood illuminator of paragraph 6 wherein at least a portion of the loop is transparent or translucent.
9. The blood illuminator of paragraph 7 wherein the shaft comprises at least one pin adapted to mate with the low voltage cable.
10. The blood illuminator of paragraph 6 wherein the loop is substantially circular, substantially oval or substantially egg shaped.
11. The blood illuminator of paragraph 2 wherein the light source has a first portion and a second portion and said first portion is angled relative to said second portion.
12. The blood illuminator of paragraph 6 wherein at least a front portion of the loop is angled relative to an end portion of the shaft.
13. The blood illuminator of paragraph 6 wherein the loop is made of a flexible material.
14. The blood illuminator of paragraph 1 further comprising a controller.
15. The blood illuminator of paragraph 12 wherein the controller is an on/off switch.
16. The blood illuminator of paragraph 12 wherein the controller automatically controls the light source.
17. The blood illuminator of paragraph 14 wherein the controller automatically controls the light source by pulsing the light, by automatically shutting off the light after a specified period of time, by automatically activating the light source at a specified time or by combinations thereof.
18. The blood illuminator of paragraph 14 wherein the controller is adapted to select the wavelength to be emitted by the light source.
19. The blood illuminator of paragraph 11 wherein the controller is a computer.
20. The blood illuminator of paragraph 17 wherein the computer is adapted to maintain and/or transmit treatment records.
21. The blood illumination system of paragraph 20 wherein the light source is a cold fusion light source.

## Claims

1. A therapeutic light treatment device comprising:
a light source emitting UV-C light, the light source having an electrical connection end;
a waterproof connector having a first end for receiving the electrical connection end from the light source;
a voltage cable having an end secured to another end of the waterproof connector, such that the voltage cable is electrically connected to the light source; and
the light source further having a closed looped end portion capable of being placed in a user's mouth and a pair of ends positioned adjacent to each other and wherein both ends are secured to the electrical connection end, and wherein positioned between the ends and the closed looped end portion is a angled section that positions the closed looped end portion downwardly.

2. A therapeutic light treatment device comprising:
a light source emitting UV-C light, the light source having an electrical connection end in communication with a power source, and wherein
the light source further having a closed looped end portion capable of being placed in a user's mouth and a pair of ends positioned adjacent to each other and wherein both ends are secured to the electrical connection end, and wherein positioned between the pair of ends and the closed looped end portion is an angled section that positions the closed looped end portion downwardly.
